(19) Europäisches Patentamt
European Patent Office
Office européen des brevets

(11) Veröffentlichungsnummer : **0 401 532 B1**

(12)

# EUROPÄISCHE PATENTSCHRIFT

(45) Veröffentlichungstag der Patentschrift :
**24.03.93 Patentblatt 93/12**

(51) Int. Cl.$^5$ : **C07C 59/185,** C07C 51/00

(21) Anmeldenummer : **90108761.9**

(22) Anmeldetag : **10.05.90**

(54) **Verfahren zur Herstellung lagerstabiler Lävulinsäure.**

(30) Priorität : **05.06.89 AT 1356/89**

(43) Veröffentlichungstag der Anmeldung :
**12.12.90 Patentblatt 90/50**

(45) Bekanntmachung des Hinweises auf die
Patenterteilung :
**24.03.93 Patentblatt 93/12**

(84) Benannte Vertragsstaaten :
**AT BE CH DE DK ES FR GB GR IT LI LU NL SE**

(56) Entgegenhaltungen :
**WO-A-80/02423**
**DE-A- 2 112 726**

(73) Patentinhaber : **Chemie Linz Gesellschaft**
**m.b.H.**
**St.Peter-Strasse 25**
**A-4021 Linz (AT)**

(72) Erfinder : **Farnleitner, Lorenz, Dr.**
**Binderlandweg 19**
**A-4020 Linz (AT)**
Erfinder : **Stückler, Hubert, Dr.**
**Mitterleitenweg 7c**
**A-4020 Linz (AT)**
Erfinder : **Kaiser, Herbert**
**Gruberstrasse 69**
**A-4020 Linz (AT)**
Erfinder : **Kloimstein, Engelbert**
**Schiferplatz 22**
**A-4070 Eferding (AT)**

(74) Vertreter : **Kunz, Ekkehard, Dr.**
**Chemie Holding AG Patentwesen St.**
**Peter-Strasse 25**
**A-4021 Linz (AT)**

**Beschreibung**

Die Erfindung betrifft ein Verfahren zur Herstellung lagerstabiler Lävulinsäure durch Verseifen von Acetylsuccinaten.

Lävulinsäure ist Ausgangsprodukt für die Herstellung organischer Chemikalien, Farbstoffe, Polymeren, pharmazeutischer Wirkstoffe und Geschmacksstoffe. Insbesondere bei der Verwendung von Lävulinsäure zur Herstellung von Polymeren, pharmazeutischen Wirkstoffen und Geschmacksstoffen werden hohe Anforderungen an Reinheit, Farbe und Stabilität der Lävulinsäure gestellt.

Es sind bereits mehrere Verfahren zur Herstellung von Lävulinsäure ausgehend von unterschiedlichen Ausgangsverbindungen bekannt.

Aus G.J. Mulder, J. prakt. Chem. 21, 219 (1840) zitiert in L.F. Wiggins, Research 3, (1950), 140, ist die Herstellung von Lävulinsäure aus Kohlehydraten durch Einwirkung von Mineralsäuren bekannt. Als Nebenprodukte entstehen bei Ausbeuten von 40 - 60 % neben Ameisensäure weitere teilweise unlösliche, teilweise tief gefärbte Nebenprodukte, die nicht vollständig abgetrennt werden können. Eine auf diese Weise hergestellte Lävulinsäure weist bereits eine deutliche braune bis rotstichige Färbung auf und dunkelt bei der Lagerung rasch nach, ist also nicht farbstabil.

Aus der DE-A 21 12 726 ist die Herstellung von Lävulinsäure ausgehend von Furfurylalkohol durch Ringspaltung mit Salzsäure oder Oxalsäure bekannt. Zur Verbesserung der Ausbeute wird bei diesem Verfahren in sehr verdünnter Lösung gearbeitet, was einen hohen Energieaufwand bei der Abtrennung des Lösungsmittels nach sich zieht. Eine auf diese Weise hergestellte Lävulinsäure zeigt jedoch bereits bei kurzer thermischer Belastung eine sehr rasche Dunkelfärbung, hat also geringe Farbstabilität.

Aus der EP-A 0 028 234 ist ein Verfahren zur Herstellung von Lävulinsäure bekannt, bei dem vorerst Furfurylalkohol in Gegenwart eines Säurekatalysators zu einem Lävulinsäureester verestert, dieser Ester in Gegenwart eines hochsiedenden Lösungsmittels durch Destillation gereinigt und anschließend in Gegenwart von Wasser und einer starken Säure hydrolysiert wird, wobei eine wäßrige Lävulinsäurelösung entsteht. Diese Lävulinsäurelösung weist eine schwache Färbung auf, die Lävulinsäure dunkelt jedoch ebenfalls bei kurzer thermischer Belastung rasch nach.

Die Herstellung von Lävulinsäure aus Furfurylalkohol ist trotz ihrer Nachteile, das bisher einzige industriell durchgeführte Verfahren.

In M. Conrad, Ber. Dt. Chem. Ges. 11, 211 (1878) und M. Conrad, Ann. 188, 1216 (1877) wird die Verseifung von Diethylacetylsuccinat mit konzentrierter Salzsäure bzw. mit Ba(OH)$_2$ oder KOH zu Lävulinsäure beschrieben. Bei der sauren Verseifung entsteht als Nebenprodukt Lävulinsäureethylester. Bei der alkalischen Verseifung kommt es zu einer Abspaltung der Acetylgruppe, sodaß als Nebenprodukt Bersteinsäure entsteht. Auf diese Weise durch Verseifung von Diethylacetylsuccinat hergestellte Lävulinsäure weist bereits nach der Isolierung durch Destillation bei geringstmöglicher Temperaturbelastung eine dunkle Färbung auf, die sich bei Lagerung rasch verschlechtert.

Es konnte nun überraschenderweise ein Verfahren zur Herstellung von Lävulinsäure ausgehend von Acetylsuccinaten gefunden werden, bei dem farbstabile Lävulinsäure in hoher Reinheit mit guter Ausbeute erhalten wird.

Gegenstand des Verfahrens ist daher ein Verfahren zur Herstellung farbstabiler Lävulinsäure durch Verseifung von Acetylsuccinaten mit wäßrigen Mineralsäuren, dadurch gekennzeichnet, daß die Ausgangsprodukte in einer Reaktorkaskade kontinuierlich im Gegenstrom mit Wasserdampf behandelt werden, wobei die Reaktion über dem Siedepunkt des bei der Reaktion entstehenden Alkohols oder über dem Siedepunkt des entstehenden wäßrigen Azeotrops durchgeführt wird.

Als Ausgangsverbindungen werden Acetylsuccinate eingesetzt, die sich von Alkoholen herleiten, deren Siedepunkt bzw. deren Siedepunkt im Azeotrop mit Wasser unter 100°C liegt. Beispiele für solche Alkohole sind Methanol, Ethanol, Propanol, i-Propanol, n-Butanol, t-Butanol. Bevorzugt wird Dimethylacetylsuccinat oder Diethylacetylsuccinat, besonders bevorzugt Dimethylacetylsuccinat eingesetzt. Als Mineralsäuren können Salzsäure oder Schwefelsäure eingesetzt werden, vorzugsweise wird wäßrige Salzsäure eingesetzt.

Zur Durchführung des Verfahrens werden die Ausgangsverbindungen vermischt, wobei das Molverhältnis von Acetylsuccinat : Mineralsäure 1 : 1 bis 7 : 1, bevorzugt 3 : 1 beträgt.

Die Ausgangsverbindungen werden im oberen Teil der Reaktorkaskade, die vorzugsweise durch eine Bodenkolonne verwirklicht wird eingebracht und mit Wasserdampf im Gegenstrom behandelt, wobei pro kg Acetylsuccinat mindestens 0,85 kg Wasserdampf eingebracht werden.

Die Verweilzeit in der Reaktorkaskade soll eine möglichst quantitative Decarboxylierung des Acetylsuccinats und Hydrolyse des entstehenden Lävulinsäureesters zu Lävulinsäure ermöglichen. Die dazu notwendige Verweilzeit ist vom Flüssigkeitsniveau auf den Böden und von der Bodenzahl abhängig. Vorzugsweise wird eine Bodenkolonne mit definiertem Flüssigkeitsniveau auf den Böden (Hold up) eingesetzt, wobei ein Leerlaufen der Böden vermieden werden soll. In der Regel sind Verweilzeiten von 30-60 min ausreichend. Kopf- und Sumpftemperatur der Reaktorkaskade werden so geregelt, daß der bei der Umsetzung entstehende Alkohol gemeinsam mit Wasser und $CO_2$ aus der Kolonne ausgetrieben wird, die Mineralsäure aber in der Kolonne bleibt, wobei zwischen Kopf- und Sumpftemperatur vorzugsweise eine Temperaturdifferenz von mindestens 10°C eingehalten wird. Bei Verwendung von Salzsäure als Mineralsäure wird vorzugsweise eine Kopftemperatur von 90 - 100°C und eine Sumpftemperatur von 110 - 140°C eingehalten.

Durch die kontinuierliche Entfernung des bei der Hydrolyse des nach der Decarboxylierung entstandenen Lävulinsäureesters entstehenden Alkohols wird der Anteil an Lävulinsäureester im Endprodukt auf minimale Mengen reduziert.

Am Boden der Kolonne wird Rohlävulinsäure abgezogen und anschließend destillativ bei möglichst geringer Temperaturbelastung gereinigt. Die dabei abgetrennte Salzsäure kann gegebenenfalls wieder in die Kolonne eingebracht werden.

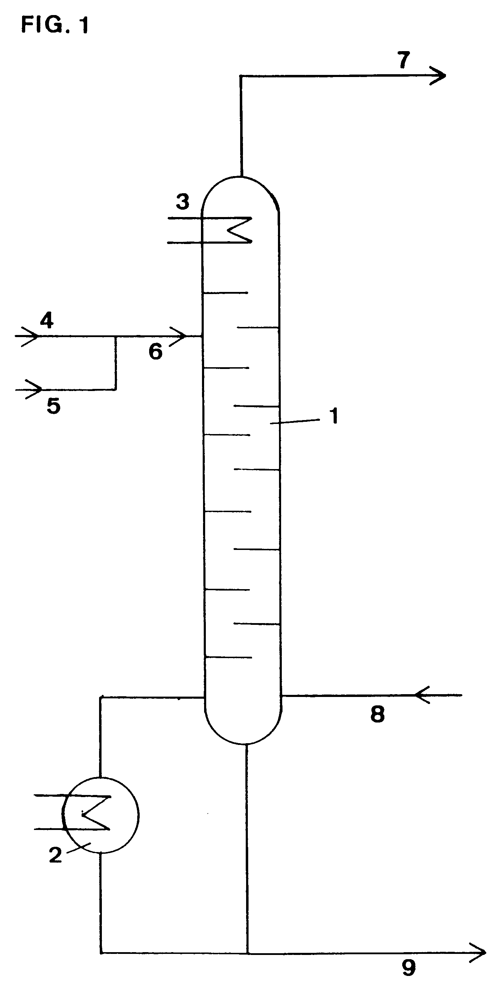

Eine bevorzugte Ausführungsform des erfindungsgemäßen Verfahrens ist in Fig. 1 dargestellt. In Fig. 1 bedeuten 1 die Reaktorkaskade, beispielsweise eine Glockenbodenkolonne, 2 den Wärmeaustauscher, 3 den Dephlegmator, 4 die Zuleitung des Acetylsuccinats, 5 die Zuleitung der Mineralsäure, 6 die Zufuhrleitung der vermischten Ausgangsverbindungen, 7 das Brüdenrohr, 8 die Wasserdampfzufuhr, 9 die Leitung zur Entnahme der Rohlävulinsäure.

Acetylsuccinat aus Leitung 4 wird mit Mineralsäure aus Leitung 5 vermischt und über Leitung 6 im oberen Teil der Kolonne bei einer Temperatur von etwa 100 eingebracht. Über Leitung 8 wird überhitzter Wasserdampf eingeblasen und nach oben durch die Kolonne geleitet.

Im Sumpf der Kolonne wird das vom untersten Boden der Kolonne ablaufende Reaktionsgemisch über einen Wärmeaustauscher 2 durch Verdampfen von Wasser aufkonzentriert. Über Leitung 9 wird die entstandene Rohlävulinsäure ausgetragen und in einer anschließenden Vakuumdestillation bei möglichst geringer Temperaturbelastung gereinigt. Am Kopf der Kolonne wird die Temperatur durch den Dephlegmator 3 so geregelt, daß die Mineralsäure nicht über das Brüdenrohr 7 gemeinsam mit dem Alkohol, Wasser und $CO_2$ ausgetragen wird, sondern überwiegend in der Rohlävulinsäure bleibt. Die über Leitung 9 abgezogene Rohlävulinsäure enthält je nach Sumpftemperatur unterschiedliche Mengen an Mineralsäure, die bei der Vakuumdestillation abgetrennt und wieder über Leitung 5 in die Kolonne eingebracht werden kann.

Nach dem erfindungsgemäßen Verfahren wird Lävulinsäure bei kurzen Verweilzeiten in hoher Ausbeute und ausgezeichneter Farbstabilität erhalten. In der Regel werden Ausbeuten von 85 - 99 % d. Theorie bezogen auf Acetylsuccinat erzielt. Die Verweilzeit beträgt im allgemeinen nur zwischen 30 und 60 Minuten, wodurch die Bildung unlöslicher oder nicht abtrennbarer, die Reinheit, Farbe und Farbstabilität beeinträchtigender Nebenprodukte vermieden wird. Die gesamte Reaktionsdauer beträgt etwa 1 - 2 Stunden.

Die erfindungsgemäß hergestellte Lävulinsäure weist nach der destillativen Reinigung eine Gardener-Farbzahl von etwa 2 nach ASTM D1544 - 8 auf, die sich auch bei starker thermischer Belastung gegenüber nach bekannten Verfahren hergestellter Lävulinsäure nur langsam und in weitaus geringerem Ausmaß verschlechtert.

Beispiel 1:

In einer in Fig. 1 dargestellten Reaktorkaskade mit 28 Glockenböden und einem Durchmesser von 300 mm wurden auf dem 23 Boden stündlich 87,5 kg Reaktionsgemisch eingebracht. Das Gemisch bestand aus 57,5 kg Dimethylacetylsuccinat und 30 kg einer 12 %igen Salzsäure. Unterhalb des untersten Bodens wurden stündlich 50 kg Wasserdampf in die Kolonne eingeblasen. Die Sumpftemperatur wurde bei 115°C gehalten. Über Kopf entwichen stündlich 84,3 kg eines Dampfgemisches, bestehend aus Methanol, Wasser $CO_2$ und etwas Salzsäure. Die Temperatur dieses durch die Leitung 5 entweichenden Gemisches wurde mittels Dephlegmator 3 auf 99 - 100°C gehalten.

Den Sumpf der Kolonne verließen stündlich 53,2 kg Rohlävulinsäure über die Leitung 9 folgender chemischer Zusammensetzung:

| | |
|---|---|
| 65,8 % | Lävulinsäure |
| 1,2 % | Lävulinsäuremethylester |
| 0,2 % | Dimethylacetylsuccinat |
| 0,5 % | Unbekannte Nebenprodukte (die mit dem eingesetzten techn. Dimethylacetylsuccinat eingebracht werden. |
| 28,0 % | Wasser |
| 4,3 % | HCl |

Die Rohlävulinsäure wurde durch fraktionierte Vakuumdestillation gereinigt, wobei stündlich 33,8 kg reine Lävulinsäure gewonnen wurden, entsprechend einer Ausbeute von 95,2 %. Die so gereinigte Lävulinsäure besaß eine Farbzahl nach Gardener von 1 - 2 und zeigte bei der Lagerung keine Farbverschlechterung.

Beispiel 2:

In der in Beispiel 1 bzw. Fig. 1 beschriebenen Apparatur wurden auf dem 23 Boden stündlich 87,5 kg/h Reaktionsgemisch eingebracht. Das Gemisch bestand aus 57,5 kg Dimethylacetylsuccinat und 30 kg Vorlaufdestillat aus der Lävulinsäure - Reindestillation, welches mit 36 %iger HCl ergänzt wurde, sodaß die HCl-Konzentrateion ca. 11 - 12 % betrug.

Diese 30 kg setzten sich aus

| | |
|---|---|
| 57,7% | Wasser |
| 23,0 % | Lävulinsäure |
| 12,0 % | HCl |
| 6,7 % | Lävulinsäuremethylester |
| 0,6 % | Methanol und unbekannte Nebenprodukte |
| | zusammen. |

Unterhalb des untersten Bodens wurden stündlich 50 kg Wasserdampf in die Kolonne eingeblasen. Die Sumpftemperatur wurde bei 115°C gehalten. Über Kopf entwichen stündlich 71,5 kg/h eines Dampfgemisches bestehend aus Methanol, Wasser, $CO_2$ und etwas Salzsäure. Die Temperatur dieses entweichenden Gemisches wird mittels Dephlegmator auf 100°C gehalten.

Den Sumpf der Kolonne verließen stündlich 66 kg Rohlävulinsäure folgender chemischer Zusammensetzung:

| | |
|---|---|
| 64,2 % | Lävulinsäure |
| 3,0 % | Lävulinsäuremethylester |
| 0,2 % | Dimethylacetylsuccinat |
| 0,4 % | Unbekannte Nebenprodukte, die mit dem eingesetzten techn. Dimethylacetylsuccinat eingebracht werden. |
| 28,0 % | Wasser |
| 4,2% | HCl |

Diese Rohlävulinsäure wurde durch fraktionierte Vakuumdestillation gereinigt, wobei stündlich 33,5 kg reine Lävulinsäure gewonnen wurden, entsprechend einer Ausbeute von 94,6 %. Die so gereinigte Lävulinsäure besaß eine Farbzahl nach Gardener von 1 - 2 und zeigte bei der Lagerung keine Farbverschlechterung.

Vergleichsbeispiel:

244,6 g Dimethylacetylsuccinat und 520 ml HCl (17 %ig) wurden solange unter Rückfluß erhitzt, bis mit dem Ende der $CO_2$-Entwicklung das Ende der Umsetzung erreicht war. Die Dauer der Reaktion betrug 5 h. Das Reaktionsgemisch wurde eingeengt und der Rückstand anschließend bei 0,013 bar destilliert. Es wurden 86 g (57 % d. Th.) Lävulinsäure erhalten.
Bp. 138 - 140°C bei 0,013 bar.

Die so erhaltene Lävulinsäure wies eine Gardener Farbzahl von 2 auf, die sich nach einmonatiger Lagerung bei Raumtemperatur auf eine Gardener Farbzahl von 6 verschlechterte.

Zur Untersuchung der Farbstabilität wurde nach Beispiel 1 hergestellte Lävulinsäure unterschiedlicher thermischer Belastung unterworfen und mit einer aus Furfurylalkohol nach DE-A 21 12 726 hergestellten Lävulinsäure (Fa. Otsuka) verglichen. Die Ergebnisse sind in Tab. 1 dargestellt:

a nach Beispiel 1 hergestellte Lävulinsäure
b aus Furfurylalkohol hergestellte Lävulinsäure (Fa. Otsuka)
c nach Vergleichsbeispiel hergestellte Lävulinsäure

Tabelle 1

Gardener Farbzahl bei unterschiedlicher thermischer Belastung

| T (°C) | 35 | | | 100 | | | 150 | | |
|---|---|---|---|---|---|---|---|---|---|
| h | a | b | c | a | b | c | a | b | c |
| 1 | 2 | 1 | 3 | 3 | 1 | 3 | 5 | 13 | 10 |
| 2 | 2 | 1 | 3 | 3 | 1 | 3 | 6 | 14 | 12 |
| 3 | 2 | 1 | 3 | 3 | 2 | 4 | 7 | 15 | 13 |
| 4 | 2 | 1 | 3 | 3 | 3 | 4 | 8 | 16 | 15 |
| 5 | 2 | 1 | 3 | 4 | 3 | 4 | 8 | 17 | 16 |
| 6 | 2 | 1 | 3 | 4 | 3 | 4 | 9 | 17 | 17 |
| 7 | 2 | 1 | 3 | 4 | 3 | 4 | 9 | 18 | 18 |
| 8 | 2 | 1 | 3 | 5 | 4 | 5 | | | |
| 9 | 2 | 1 | 3 | 5 | 4 | 6 | | | |
| 24 | 2 | 1 | | 5 | 6 | | | | |

**Patentansprüche**

1. Verfahren zur Herstellung farbstabiler Lävulinsäure durch Verseifung von Acetylsuccinaten mit wäßrigen Mineralsäuren, dadurch gekennzeichnet, daß die Ausgangsprodukte in einer Reaktorkaskade kontinuierlich im Gegenstrom mit Wasserdampf behandelt werden, wobei die Reaktion über dem Siedepunkt des bei der Reaktion entstehenden Alkohols oder über dem Siedepunkt des entstehenden wäßrigen Azeotrops durchgeführt wird.

2. Verfahren nach Anspruch 1, dadurch gekennzeichnet, daß als Acetylsuccinate jene Diester der Acetylbernsteinsäure verwendet werden, die sich von Alkoholen herleiten, deren Siedepunkt oder deren Siedepunkt im Azeotrop mit Wasser unter 100°C liegt.

3. Verfahren nach Anspruch 2, dadurch gekennzeichnet, daß als Acetylsuccinat Dimethylacetylsuccinat eingesetzt wird.

4. Verfahren nach einem der Ansprüche 1 bis 3, dadurch gekennzeichnet, daß als wäßrige Mineralsäure wäßrige Salzsäure eingesetzt wird.

5. Verfahren nach einem der Ansprüche 1 bis 4, dadurch gekennzeichnet, daß das Molverhältnis Acetylsuccinat: HCl 1 : 1 bis 7 : 1 beträgt.

6. Verfahren nach Anspruch 5, dadurch gekennzeichnet, daß das Molverhältnis Acetylsuccinat: HCl 3 : 1 beträgt.

7. Verfahren nach einem der Ansprüche 1 bis 6, dadurch gekennzeichnet, daß pro kg Acetylsuccinat mindestens 0,85 kg Wasserdampf eingesetzt werden.

8. Verfahren nach einem der Ansprüche 1 bis 7, dadurch gekennzeichnet, daß die Verweilzeit in der Reaktorkaskade 30 - 60 min beträgt.

9. Verfahren nach einem der Ansprüche 1 bis 8, dadurch gekennzeichnet, daß im Sumpf der Reaktorkaskade eine Temperatur zwischen 110 - 140°C und am Kopf der Reaktorkaskade eine Temperatur zwischen 90 und 100°C eingehalten wird, wobei eine Temperaturdifferenz zwischen Kopf und Sumpf von mindestens 10°C aufrecht erhalten wird.

**Claims**

1. Process for the preparation of colour-stable levulinic acid by saponification of acetylsuccinates with aqueous mineral acids, characterized in that the starting products are continuously treated with steam in counter-current in a reactor cascade, the reaction being carried out above the boiling point of the alcohol being formed in the reaction or above the boiling point of the aqueous azeotrope being formed.

2. Process according to Claim 1, characterized in that the acetylsuccinates used are those diesters of acetylsuccinic acid which are derived from alcohols whose boiling point or whose boiling point in the azeotrope with water is below 100°C.

3. Process according to Claim 2, characterized in that the acetylsuccinate used is dimethyl acetylsuccinate.

4. Process according to one of Claims 1 to 3, characterized in that the aqueous mineral acid used is aqueous hydrochloric acid.

5. Process according to one of Claims 1 to 4, characterized in that the acetylsuccinate : HCl molar ratio is 1 : 1 to 7 : 1.

6. Process according to Claim 5, characterized in that the acetylsuccinate : HCl molar ratio is 3 : 1.

7. Process according to one of Claims 1 to 6, characterized in that at least 0.85 kg of steam is used per kg of acetylsuccinate.

8. Process according to one of Claims 1 to 7, characterized in that the residence time in the reactor cascade is 30-60 minutes.

9. Process according to one of Claims 1 to 8, characterized in that a temperature between 110 and 140°C is maintained in the bottom of the reactor cascade and a temperature between 90 and 100°C is maintained at the top of the reactor cascade, a temperature difference of at least 10°C being maintained between the top and bottom.

**Revendications**

1. Procédé de préparation d'acide lévulique stable en coloration par saponification de succinates d'acétyle avec des acides minéraux aqueux, caractérisé en ce qu'on traite les produits de départ dans une cascade de réacteurs de manière continue, dans un contre courant de vapeur d'eau, la réaction étant conduite au-dessus du point d'ébullition de l'alcool produit au cours de la réaction ou bien au-dessus du point d'ébullition de l'azéotrope aqueux produit.

2. Procédé selon la revendication 1, caractérisé en ce qu'on utilise comme succinates d'acétyle les diesters de l'acide acétylsuccinique, qui sont obtenus à partir des alcools dont le point d'ébullition ou bien le point d'ébullition de l'azéotrope avec l'eau est situé au-dessous de 100°C.

3. Procédé selon la revendication 2, caractérisé en ce qu'on utilise comme succinate d'acétyle le succinate de diméthylacétyle.

4. Procédé selon l'une des revendications 1 à 3, caractérisé en ce que l'acide minéral aqueux est l'acide chlorhydrique aqueux.

5. Procédé selon l'une des revendications 1 à 4, caractérisé en ce que le rapport molaire succinate d'acétyle : HCl est de 1:1 à 7:1.

6.  Procédé selon la revendication 5, caractérisé en ce que le rapport molaire succinate d'acétyle : HCl est de 3:1.

7.  Procédé selon l'une des revendications 1 à 6, caractérisé en ce qu'on utilise au moins 0,85 kgs de vapeur d'eau par kg de succinate d'acétyle.

8.  Procédé selon l'une des revendications 1 à 7, caractérisé en ce que le temps de contact dans la cascade de réacteurs est de 30 à 60 mn.

9.  Procédé selon l'une des revendications 1 à 8, caractérisé en ce qu'on maintient en queue de la cascade de réacteurs une température comprise entre 110 et 140°C et en tête de la cascade de réacteurs, une température comprise entre 90 et 100°C, une différence de température d'au moins 10°C étant maintenue entre ces deux points.

FIG. 1